(19) Europäisches Patentamt
European Patent Office
Office européen des brevets

(11) Publication number : **0 219 823 B1**

# (12) EUROPEAN PATENT SPECIFICATION

(45) Date of publication of patent specification : **16.01.91 Bulletin 91/03**

(51) Int. Cl.$^5$ : **C07C 17/04,** C07C 19/08

(21) Application number : **86114359.2**

(22) Date of filing : **16.10.86**

## (54) Process for the preparation of hydrofluoroalkanes.

(30) Priority : **21.10.85 IT 2256485**

(43) Date of publication of application : **29.04.87 Bulletin 87/18**

(45) Publication of the grant of the patent : **16.01.91 Bulletin 91/03**

(84) Designated Contracting States : **DE FR GB**

(56) References cited :
**EP-A- 0 196 630**
**GB-A- 1 420 428**
**GB-A- 1 468 543**
**US-A- 4 377 715**
**Patent Abstracts of Japan, unexamined applications, field C, vol. 7, no. 122, May 26, 1983.The Patent Office Japanese Government,page 96 C 168**
**The Journal of Organic Chemistry, vol. 28, February 1963, Easton Rausch et al. "The addition of fluorine to halogenated olefins by means of metal fluorides" pages 494-497**

(73) Proprietor : **AUSIMONT S.r.l.**
**Foro Buonaparte 31**
**I-20121 Milano (IT)**

(72) Inventor : **Gervasutti, Claudio**
**2/4, via P. Sarpi**
**I-30175 Mestre Venezia (IT)**
Inventor : **Conte, Lino**
**15, via Padre Leopoldo**
**I-35028 Piove di Sacco Padova (IT)**
Inventor : **Gambaretto, Gian Paolo**
**7, via Torino**
**I-35142 Padova (IT)**

(74) Representative : **Barz, Peter, Dr. et al**
**Patentanwälte Dipl.-Ing. G. Dannenberg Dr. P. Weinhold, Dr. D. Gudel Dipl.-Ing. S. Schubert, Dr. P. Barz Siegfriedstrasse 8**
**D-8000 München 40 (DE)**

**EP 0 219 823 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

The present invention relates to a process for the preparation of hydro(halo)fluoroalkanes starting from the respective hydro(halo)alkenes.

Hydrofluoroalkanes may be prepared by reduction with $LiAlH_4$ (Ber. 97(7) 1964), or by U.V. irradiation of halofluoroalkanes in the presence of an alcohol (Czech.136, 735). However, none of these processes can be applied in industrial operations because of the high costs of the reactants or due to the difficulties relating to the equipment required for similar treatments in case of $LiAlH_4$ or to the use of U.V. light. Moreover, the process of reduction by U.V. is limited, in that only a few specific compounds mono- or dihydrogenated on the same carbon atom can be obtained by it.

In fact, in said process, the introduction of the first hydrogen atom into the molecule can be relatively simple, even if the reaction time is of the order of twenty hours but the introduction of the second hydrogen atom into the molecule is more difficult than that of the first, and no compounds di-hydrogenated on adjacent carbon atoms are obtained.

Such fluorination processes as electrofluorination carried out on hydrocarbons (J.Electrochem. Soc.95, 47, 1949) are also known but these processes are burdensome as regards the equipment and from the energy viewpoint and, above all, fragmentation occurs as a side reaction. Fluorinations of hydrocarbons carried out with elemental fluorine, generally diluted with nitrogen, in the gaseous phase over a Cu/Ag catalyst (Ind.Eng.Chem. 39,290 (1947)) are also known. These processes are, however, also very limited, in that they lead to the formation of side reaction by-products such as high amounts of fragmentation products or dimers and trimers.

Fluorinating halogenated olefins with $CoF_3$ is known as well (J.Org.Chem. 28,494 (1963)), but side reactions as intramolecular H/fluorine, halogen/fluorine substitutions occur.

Thus, by this process, the selective preparation of alkanes from olefins is not possible.

Furthermore, a fluorination process of this type requires particularly sophisticated equipment capable of withstanding the fluorine used to regenerate $CoF_3$ at a temperature around 300°C.

There was, thus, a need to produce, by a process easily accomplishable on an industrial scale, saturated compounds belonging to the class of hydro(halo)fluoroalkanes, both monohydrogenated and containing H atoms on adjacent C atoms.

It was also necessary to have available a fluorination process by which the by-product dimers, trimers, and addition polymers could be kept at a minimum and, at the same time, the simultaneous substitution by fluorine of the hydrogen or halogen atoms of the starting product kept at very low percentages.

It has now surprisingly been found that, by carrying out the fluorination reaction on hydro(halo)olefins with elemental fluorine at low temperatures of below 0°C, the fluorine addition to the double bond is preferred to substitution of the hydrogen atoms and/or of the halogen atoms different from fluorine contained in the olefin, the substituion products being contained in very low percentages. The formation of the addition dimer products is, moreover, very low while not even traces of the trimers or polymers are formed.

Thus, the object of the present invention is a process for the fluorination in the liquid phase with elemental fluorine at a temperature below 0°C of hydrohaloalkenes or of hydroalkenes having the following general formulae :

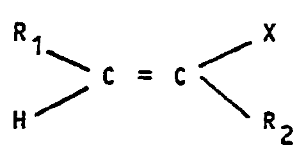

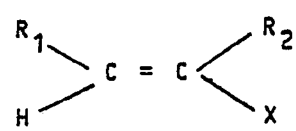

wherein :

X = H, F, Cl, Br

$R_1$, $R_2$ = H, F, Cl, Br, $C_1$-$C_3$-alkyl or -alkoxy, wherein the H atoms can also be completely substituted by such halogens as Cl or F.

The fluorination process according to the present invention is carried out in the liquid phase at temperatures of from 0°C to –100°C, preferably of from –70°C to –85°C, the liquid phase consisting of the reactant and the reaction products. Solvents present in an amount of from 1 to 20 parts, preferably 4 to 10 parts by weight (w/w) per each part of hydro(halo)olefin, can also be used. Suitable solvents are completely fluorinated and completely chlorofluorinated- alkanes which are liquid at the reaction temperature and inert under the fluorination conditions. Preferred solvents are $CFCl_3$, $CF_2Cl_2$ and $CF_2Cl$-$CF_2Cl$.

The elemental fluorine is preferably used diluted with inert gases, such as nitrogen, at an inert gas/$F_2$ molar ratio of from 5 to 15.

The reaction can be carried out under atmospheric pressure or under reduced pressure.

The process of the present invention allows the desired product to be obtained with a high selectivity and, at the same time, a high conversion of the starting product is obtained.

A further advantage of the process of the invention is that the fluorination of olefins is made possible with a relatively simple process, and with higher yields than in the processes of the prior art as mentioned above.

The products which can be obtained by the process of the invention are advantageously used as anaesthetics or, after suitable dehalogenation or dehydrohalogenation by known processes, as comonomers in the preparation of fluorinated polymers.

The following examples serve to illustrate the invention. Unless stated otherwise, in the examples parts and percentages are by weight.

EXAMPLE 1

100 g of 1,2-dichloroethylene (30/70 by weight cis/trans isomer mixture) and 1000 g of $CCl_3F$ are charged to a cylindrical reactor of Algoflon R having an inner diameter of 85 mm and a volume of 1000 ml and kept at a controlled temperature of −75°C.

The elemental fluorine, diluted to a molar ratio of 1 part with 9 parts of nitrogen, is continuously introduced over a period of 8 hours up to a total amount of 1 mol.

At the end of the test 1130 g of raw reaction product is collected and washed with an aqueous solution containing 5% by weight of NaOH. It is then thoroughly dried over $CaCl_2$, and subjected to distillation to separate the product from the solvent. The conversion into reaction products is 73 % relative to the CHCl=CHCl supplied and the yield of CHClF-CHClF is 72 % relative to the reacted matter.

EXAMPLE 2

Example 1 is repeated by introducing an amount of 1.5 mol of $F_2$ over a period of 8 hours.

The conversion is 80 % and the yield is 74 %.

EXAMPLE 3

100 g of 1,2-dichloroethylene (30/70 w/w cis/trans isomer mixture) and 1000 g of $CCl_3F$ are charged to the reactor of Example 1 which is kept at a controlled temperature of −60°C.

Elemental fluorine, diluted to a molar ratio of 1 part of $F_2$ to 9 parts of nitrogen, is continuously fed or introduced over a period of 8 hours up to a total amount of 1 mol of fluorine.

At the end of the test, 1110 g of raw reaction product are collected and purified as described in example 1. The conversion is 80 % and the yield of CHClF-CHClF is 63 %.

EXAMPLE 4

100 g of 1,2-dichloroethylene (isomeric cis/trans ratio = 30/70 w/w) and 1000 g of $CCl_3F$ are charged to the reactor of example 1 which is kept at a controlled temperature of −80°C.

Elemental fluorine, diluted to a molar ratio of 1 part of $F_2$ to 9 parts of nitrogen, is continuously introduced over a period of 8 hours up to a total amount of 1 mol of fluorine.

At the end of the test, 1130 g of raw reaction product is collected. The conversion is 68 % and the yield of CHClF-CHClF is 74 %.

EXAMPLE 5

100 g of 1,2-dichloroethylene (100 % of cis isomer) and 1000 g of $CCl_3F$ are charged to the reactor of Example 1 which is kept at a controlled temperature of −75°C. Elemental fluorine, diluted to a molar ratio of 1 part of $F_2$ to 15 parts of nitrogen, is continuously introduced over a period of 8 hours up to a total amount of 1 mol of fluorine.

At the end of the test, 1120 g of raw reaction product are collected. The conversion is 75 % and the yield of CHClF-CHClF is 70 %.

EXAMPLE 6

100 g of 1,2-dichloroethylene (100 % of trans isomer) and 1000 g of $CCl_3F$ are charged to the reactor of example 1 which is kept at a controlled temperature of −75°C

Elemental fluorine, diluted to a molar ratio of 1 part of $F_2$ to 9 parts of nitrogen, is continuously introduced over a period of 8 hours up to a total amount of 1 mol of fluorine.

At the end of the test, 1125 g of raw reaction product are collected. The conversion is 70 % and the yield of CHClF-CHClF is 74 %.

EXAMPLE 7

100 g of trichloroethylene and 1000 g of $CCl_3F$ are charged to the reactor of Example 1 which is kept at a controlled temperature of −75°C. Elemental fluorine, diluted to a molar ratio of 1 part of $F_2$ to 15 parts of nitrogen is continuously introduced over a period of 8 hours up to a total amount of 1 mol of fluorine.

At the end of the test, 1135 g of raw reaction product are collected. The conversion is 79 % and the yield of $CCl_2F$-CHClF is 93 %.

## Claims

1. A fluorination process comprising the addition of fluorine to the double bond of an organic compound of the following general formulae :

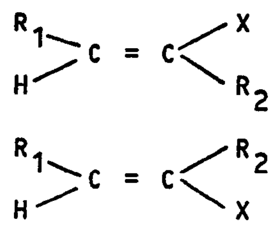

wherein :
X = H, F, Cl, Br
$R_1$, $R_2$ = H, F, Cl, Br, $C_1$-$C_3$-alkyl or -alkoxy, wherein the H atoms can also be completely substituted by such halogens as Cl or F,
characterized in that fluorination is carried out in the liquid phase using elemental fluorine at temperatures of from 0°C to −100°C.

2. A process according to claim 1, wherein the reaction temperature is from −70°C to −85°C.

3. A process according to claim 1 or 2, wherein the liquid phase is constituted by the reactant and the reaction products, in the form of a mixture with a perhalogenated inert solvent.

4. A process according to claim 3, wherein the solvent is used in an amount of from 1 to 20 parts by weight per each part of reactant.

5. A process according to any one of claims 1-4, wherein the elemental fluorine used is diluted with inert gases in a molar ratio of inert gas/$F_2$ of from 5 to 15.

## Revendications

1. Procédé de fluoration comprenant l'addition de fluor à la double liaison d'un composé organique de formules générales suivantes :

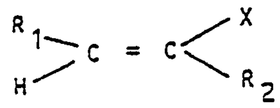

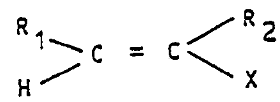

dans lesquelles : X = H, F, Cl, Br $R_1$, $R_2$ = H, F, Cl, Br, un radical alkyle ou alcoxy en $C_1$-$C_3$, où les atomes d'hydrogène peuvent également être complètement substitués par des halogènes tels que Cl ou F, caractérisé en ce que la fluoration est mise en oeuvre en phase liquide à l'aide de fluor élémentaire à des températures de 0°C à −100°C.

2. Un procédé selon la revendication 1, dans lequel la température de réaction est comprise entre −70°C et −85°C.

3. Un procédé selon la revendication 1 ou 2, dans lequel la phase liquide est constituée par le réactif et les produits de réaction, sous la forme d'un mélange avec un solvant perhalogéné inerte.

4. Un procédé selon la revendication 3, dans lequel le solvant est utilisé en une quantité de 1 à 20 parties en poids par partie du réactif.

5. Un procédé selon l'une quelconque des revendications 1 à 4, dans lequel le fluor élémentaire utilisé est dilué avec des gaz inertes dans un rapport molaire gaz inerte/$F_2$ compris entre 5 et 15.

## Ansprüche

1. Fluorierungsverfahren, umfassend die Addition von Fluor an die Doppelbindung einer organischen Verbindung der folgenden allgemeinen Formeln :

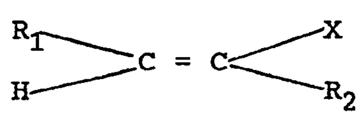

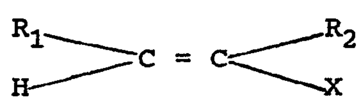

worin : X = H, F, Cl, Br $R_1$, $R_2$ = H, F, Cl, Br, $C_1$-$C_3$-Alkyl oder -Alkoxy, worin die H-Atome auch vollständig durch solche Halogene wie Cl oder F substituiert sein können, dadurch gekennzeichnet, daß die Fluorierung in flüssiger Phase unter Verwendung von elementarem Fluor bei Temperaturen von 0°C bis −100°C durchgeführt wird.

2. Verfahren nach Anspruch 1, worin die Reaktionstemperatur −70°C bis −85°C ist.

3. Verfahren nach Anspruch 1 oder 2, worin die flüssige Phase von dem Reaktanten und den Reaktionsprodukten in Form einer Mischung mit einem perhalogenierten inerten Lösungsmittel gebildet wird.

4. Verfahren nach Anspruch 3, worin das Lösungsmittel in einer Menge von 1 bis 20 Gewichtsteilen pro einem Teil Reaktant verwendet wird.

5. Verfahren nach irgendeinem der Ansprüche 1 - 4, worin das verwendete elementare Fluor mit inerten Gasen in einem Molverhältnis von Inertgas/$F_2$ von 5 bis 15 verdünnt wird.